## Europäisches Patentamt

## European Patent Office

## Office européen des brevets

(11) Numéro de publication: **0 147 253**
**B1**

(12)

# FASCICULE DE BREVET EUROPÉEN

(45) Date de publication du fascicule du brevet:
07.02.90

(51) Int. Cl. ⁵: **A 61 B 5/02, A 61 B 5/04**

(21) Numéro de dépôt: **84402153.5**

(22) Date de dépôt: **25.10.84**

(54) Appareil portatif de surveillance de l'activité cardiaque.

(30) Priorité: 10.11.83 FR 8317874

(43) Date de publication de la demande:
03.07.85 Bulletin 85/27

(45) Mention de la délivrance du brevet:
07.02.90 Bulletin 90/06

(84) Etats contractants désignés:
AT BE CH DE GB IT LI LU NL SE

(56) Documents cité:
GB-A-2 034 046
GB-A-2 070 438
US-A-4 129 125
US-A-4 239 046
US-A-4 318 412
US-A-4 353 372
US-A-4 367 752

ELECTRO MEDICA, vol. 39, no. 4, 1971, page 157, Erlangen, DE; G. HENKE: "Vereinfachte und sichere Elektrodenanlage in der Reizstrombehandlung durch Appliserv"

(73) Titulaire: **Ascher, Gilles**
**20bis, Boulevard du Général Leclerc**
**F-92200 Neuilly (FR)**
Titulaire: **Coustenoble, Jean-Pierre**
**11, rue Charcot**
**F-92800 Puteaux (FR)**
Titulaire: **Fournial, Jean-Franfois**
**La Sauvagère Route de Parinier**
**F-72560 Change (FR)**

(72) Inventeur: **Ascher, Gilles**
**20bis, Boulevard du Général Leclerc**
**F-92200 Neuilly (FR)**
Inventeur: **Coustenoble, Jean-Pierre**
**11, rue Charcot**
**F-92800 Puteaux (FR)**
Inventeur: **Fournial, Jean-Franfois**
**La Sauvagère Route de Parinier**
**F-72560 Change (FR)**

(74) Mandataire: **Rodhain, Claude**
**Cabinet Claude Rodhain 30, rue la Boétie**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dîpposition (Art. 99(1) Convention sur le brevet européen).

## Description

La présente invention concerne des appareils portatifs destinés à surveiller l'activité cardiaque en toutes circonstances, sans l'assitance d'un spécialiste.

On a déjà conçu des appareils qui comportent un boîtier qui est muni de deux électrodes de prélèvement d'électrocardiogrammes et qui contient essentiellement un dispositif électronique de traitement qui fournit un enregistrement d'électrocardiogrammes qui sera ensuite transmis au médecin traitant, et/ou la valeur du rythme cardiaque de la personne considérée.

Un appareil de ce type est essentiellement constitué par un boîtier dont le format est celui d'un paquet de cigarettes et qui porte deux électrodes extérieures sur lesquelles on applique tout ou partie d'une main afin d'enregistrer un courant d'électrocardiogramme entre les deux bras. Ces appareils permettent aux patients d'effectuer un enregistrement d'électrocardiogrammes au moment même où ils ont un malaise, ce qui est particulièrement important pour les patients qui présentent des symptômes à fréquence faible. L'électrocardiogramme ainsi enregistré est mis sous forme numérique et est mémorisé dans une mémoire de manière à pouvoir être lu ultérieurement par le médecin spécialiste.

Bien que ces appareils soient portatifs, ils ne sont pas à disposition immédiate puisqu'il faut généralement sortir l'appareil d'une poche, repérer les électrodes et y porter les mains; cette opération, bien que simple, peut demander beaucoup de temps dans l'affolement qui suit l'apparition d'un malaise cardiaque et il est possible que l'on enregistre un électrocardiogramme qui ne soit pas caractéristique du fait qu'il est trop postérieur à l'apparition du malaise. Par ailleurs, un tel appareil n'est pas toujours disponible, par exemple parce qu'il est resté dans un vêtement que l'on a quitté ou parce que les vêtements portés par le patient ne comportent pas de poche, ce qui est le cas en particulier de certaines tenues estivales ou de sport.

Par ailleurs, du fait que l'enregistrement s'effectue entre les deux mains, il comporte des parasites ou artéfacts que l'on ne peut éliminer, ce qui nuit à la qualité de l'électrocardiogramme enregistré.

On connaît également, par le brevet aux E.U.A. No. 4 129 125, un appareil portatif pour la surveillance des signes témoignant de l'état de santé d'un patient tels que sa température, sa respiration, son pouls, et le relevé d'un électrocardiogramme. Cet appareil, portatif, de surveillance de l'activité cardiaque, comporte, en vue du relevé d'un électrocardiogramme, deux électrodes branchées dans un boîtier renfermant un dispositif électronique de traitement des signaux enregistrés à partir de l'activité du coeur; ces électrodes sont séparées et affectées à deux parties distinctes du corps du patient, et plus particulièrement de la poitrine de celui-ci; l'une des électrodes est apparente sur l'une des faces du boîtier pour entrer en contact avec une première zone du corps du patient tandis que l'autre électrode est indépendante du boîtier pour entrer en contact avec une autre zone; cette autre électrode est branchée électriquement dans le boîtier de la première par un conducteur de liaison porté par un organe de fixation de l'appareil au corps du patient constitué par une ceinture élastique destinée à enserrer ce corps.

La présente invention se propose de remédier à ces inconvénients. Elle a pour objet un appareil portatif de surveillance de l'activité cardiaque, comportant deux électrodes branchées dans un boîtier renfermant un dispositif électronique de traitement des signaux enregistrés à partir de l'activité diastolique et systolique du coeur, lesdites électrodes étant séparées et affectées à deux parties distinctes de la région cardiaque du buste du patient, l'une desdites électrodes étant apparente sur l'une des faces du boîter pour entrer en contact avec une première zone du buste du patient tandis que l'autre électrode est indépendante de ce même boîtier pour entrer en contact avec une seconde zone du dit buste, ladite autre électrode étant branchée électriquement dans ledit boîtier par un conducteur de liaison, caractérisé en ce que ledit conducteur de liaison est constitué d'un lien souple en forme de boucle qui sert simultanément pour la suspension de l'appareil sur le corps du patient, ledit boîtier et ladite électrode étant fixés en des points sensiblement opposés de ladite boucle.

Dans l'appareil selon l'invention, au moins l'une des électrodes reste en contact permanent avec le buste du patient et l'autre électrode peut être rapidement mise en contact avec une autre partie du buste du patient pour obtenir l'enregistrement d'un signal de l'activité diastolique et systolique du coeur du patient. Cette opération est particulièrement rapide puisqu'il suffit de plaquer la seconde électrode sur le buste pour déclencher l'enregistrement.

Par ailleurs, les deux électrodes étant en contact avec deux zones du buste du patient dans la région cardiaque, on obtient un signal qui comporte très peu de parasites et qui est de qualité équivalente aux signaux enregistrés par des appareils d'enregistrement d'électrocardiogrammes fixes tels que ceux utilisés normalement dans les hôpitaux ou les cabinets de médecins.

Selon un mode de réalisation de l'invention, le conducteur électrique est constitué d'un lien souple en forme de boucle destiné à être passé autour du cou du patient, les deux électrodes étant fixées en des points sensiblement opposés de ladite boucle formant collier, l'une des électrodes étant appliquée sous la nuque du patient, l'autre électrode, portée par le boîtier à la manière d'un pendentif, étant en appui sur la poitrine du patient.

Dans ce cas, l'électrode extérieur au boîtier est en contact permanent avec la nuque du patient et l'autre électrode peut être plaquée très rapidement sur la poitrine en vue d'un enregistrement lors de l'apparition d'un malaise. Il y a lieu de noter que le geste de porter la main à la poitrine est un geste naturel lorsque l'on ressent une douleur et que par conséquent, l'enregistrement de l'électrocardiogramme sera obtenu de manière pratiquement instinctive. L'électrode extérieure au boîtier est maintenue en contact permanent avec la peau sous l'effet de la force exercée sur le lien souple par le poids du boîtier.

Par ailleurs, l'utilisation d'un lien souple permet d'adapter l'appareil à la morphologie de chaque patient qui peut, lors de l'apparition d'un malaise, plaquer le boîtier en une zone particulièrement favorable de la poitrine.

De préférence les deux électrodes sont souples et déformables et réalisées en une matière conductrice du type à mailles ou à tresses. Cette configuration permet d'obtenir un bon contact avec la peau du patient même dans le cas où l'électrode considérée est en contact avec une zone présentant une forte pilosité.

De préférence au moins l'une des deux électrodes comporte un organe d'accouplement avec une électrode auxiliaire fixée sur la peau du patient, ces deux électrodes comportant au moins un ensemble d'organes d'accouplement mécanique et de liaison électrique complémentaires du type "bouton-pression" à clipsage à organe mâle et siège femelle.

Cette disposition permet de faire fonctionner l'appareil selon l'invention en surveillance permanente ou "sentinelle", le dispositif électronique de traitement comportant alors une mémoire tournante; dans ce cas, l'enregistrement est effectué en permanence et l'actionnement du boîtier commande l'enregistrement de signaux s'étant produit avant et après ladite commande, de manière à bien couvrir l'instant d'apparition du malaise.

On peut également prévoir un dispositif auxiliaire en forme de boîtier plat comportant sur une de ses faces un dispositif d'accouplement mâle du type précité et sur la face opposée un dispositif d'accouplement femelle; ce dispositif auxiliaire est par ailleurs relié à des électrodes collées en permanence sur différents endroits du buste du patient. Cette disposition permet d'obtenir différents types d'enregistrement à partir de plusieurs électrodes prises deux par deux et dans ce cas le signal enregistré est pratiquement identique à celui obtenu dans une installation fixe d'enregistrement d'électrocardiogrammes.

D'autres caractéristiques et avantages de l'invention ressortiront de la description qui suit, faite à titre illustratif en se référant aux dessins ci-annexés sur lesquels:

- les fig. 1 et 2 représentent un mode de réalisation de l'appareil selon l'invention en place sur un patient;
- la fig. 3 représente la face arrière du boîtier avec son électrode;
- la fig. 4 représente en coupe selon la coupe III - III de la fig. 2 le boîtier muni d'une électrode auxiliaire;

- la fig. 5 représente toujours en coupe, un boîtier muni d'un dispositif auxiliaire et d'une électrode auxiliaire;
- la fig. 6 est le schéma synoptique d'un exemple de réalisation du dispositif électronique de traitement;
- la fig. 7 est le schéma synoptique d'un exemple de réalisation du circuit électronique compris dans le dispositif auxiliaire, et
- la fig. 8 représente une possibilité d'implantation des électrodes auxiliaires.

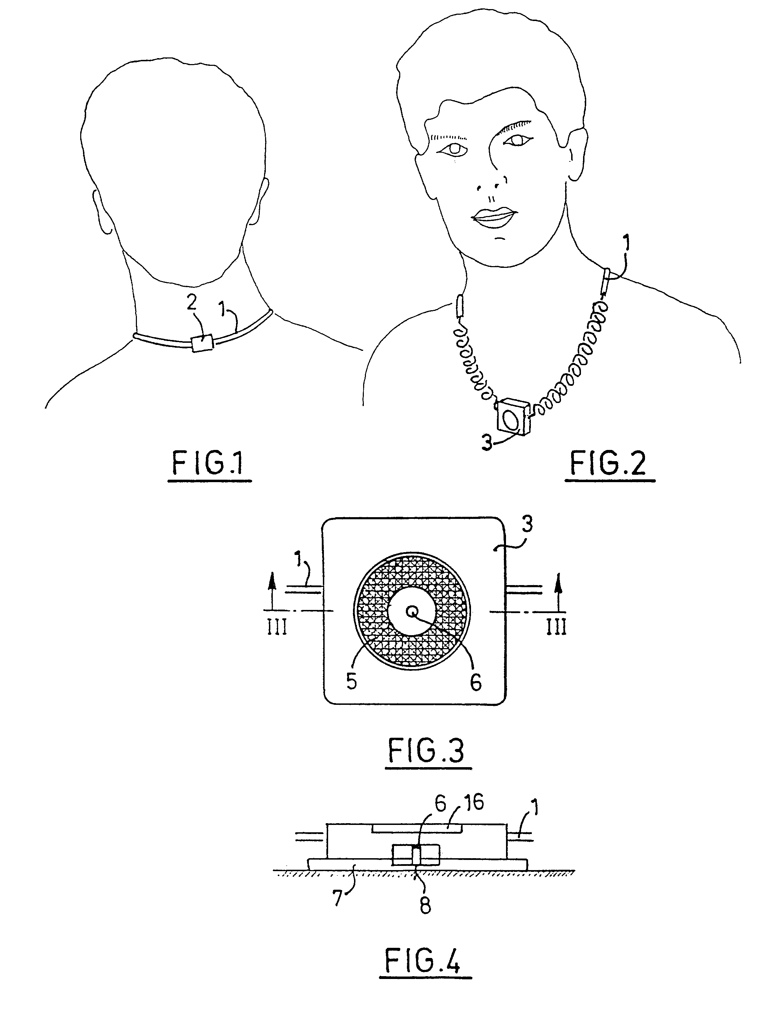

Selon l'invention, les deux électrodes de l'appareil portatif de surveillance de l'activité cardiaque sont séparées, l'une d'entre elles étant portée par un boîtier renfermant le dispositif électronique de traitement et l'autre électrode étant reliée audit boîtier par un lien conducteur qui sert également à la suspension de l'appareil sur le corps du patient. Selon le mode de réalisation qui a été représenté sur les fig. 1 et 2, le lien ou conducteur électrique 1 est constitué d'un lien souple en forme de boucle qui est passé autour du cou du patient et qui porte d'une part une électrode extérieure 2 qui est appliquée sur la nuque du patient, et, d'autre part, le boîtier 3 qui renferme le dispositif électronique de traitement et qui est muni sur une de ses faces d'une électrode. Le lien 1 peut par exemple être du type spiralé, de manière à présenter une élasticité suffisante pour pouvoir amener le boîtier 3 en contact avec toutes zones désirées de la poitrine du patient.

L'électrode extérieure 2 est constituée par un petit boîtier plat qui comporte une électrode sur la face en contact avec la peau; de manière analogue, le boîtier 3 comporte sur sa face 4 en regard du buste du patient, une électrode 5. Ces deux électrodes sont avantageusement réalisées en une matière conductrice du type à mailles ou à tresses, ce qui permet d'obtenir une électrode souple et déformable qui s'adapte au profil de la zone du buste du patient sur laquelle elle est appliquée. Ce mode de réalisation de l'électrode permet donc d'obtenir un bon contact même dans le cas où la zone où l'électrode est appliquée présente un système pileux important.

De préférence au moins l'une des deux électrodes comporte un organe d'accouplement mécanique et de liaison électrique destiné à son raccordement avec une électrode auxiliaire collée en permanence sur le buste du patient, cette électrode auxiliaire comportant un organe d'accouplement complémentaire.

Dans le mode de réalisation représenté, cet organe d'accouplement mécanique est constitué par un organe à clipsage du type "bouton-pression", l'électrode principale de l'appareil étant munie de la partie femelle de cet accouplement et l'électrode auxiliaire de la partie mâle. Ceci est représenté en particulier sur les fig. 3 et 4, où l'on voit la partie femelle 6 du bouton-pression qui est fixée sans présenter de relief sur la face 4 du boîtier 3 et l'électrode auxiliaire 7 est munie d'une partie mâle 8 coopérant avec la partie femelle 6. Avantageusement dans ce cas, l'électrode auxiliaire 7 est encollée des deux côtés de manière que le boîtier 3 adhère bien à la peau et qu'il n'ait pas tendant à entraîner l'électrode vers le bas. L'électrode auxiliaire 2 peut également être munie d'un tel dispositif de raccordement mécanique du type "bouton-pression". Les électrodes auxiliaires peuvent être des électrodes ambulatoires dites de HOLTER.

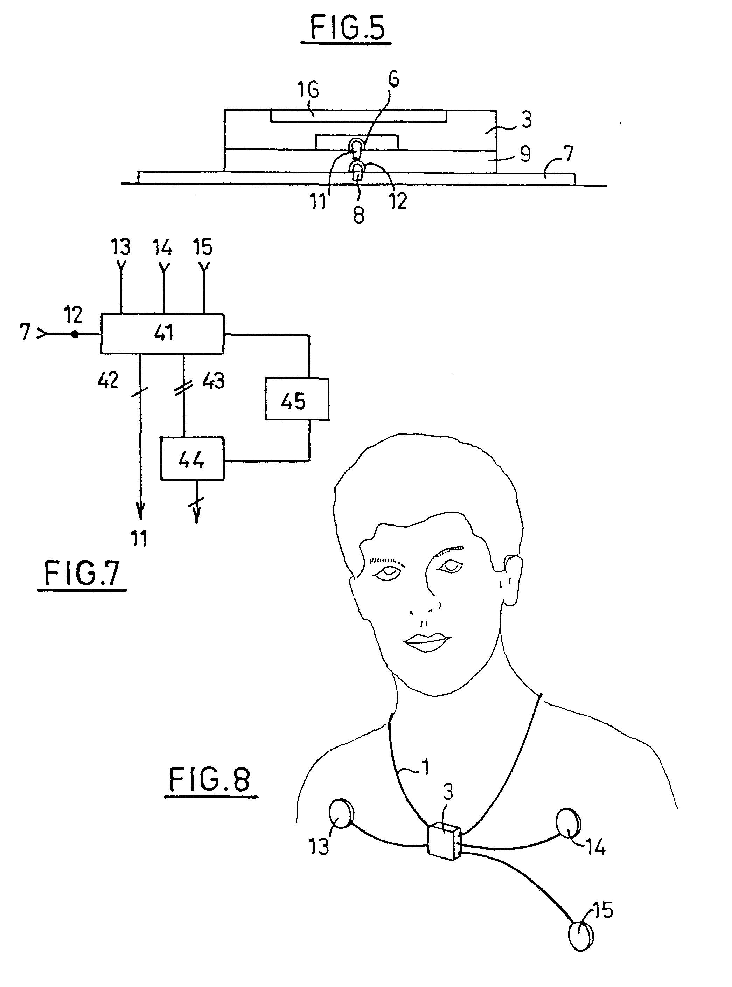

Sur la fig. 5 on a représenté une variante de l'invention selon laquelle on prévoit un dispositif auxiliaire 9 qui a la forme d'un boîtier très plat et qui comporte sur une de ses faces un élément d'accouplement mâle 11 et sur la face opposée un élément d'accouplement femelle 12. Comme on peut le voir sur la fig. 5, ce dispositif auxiliaire est disposé entre le boîtier 3 et l'électrode auxiliaire 7 et, comme cela est représenté à la fig. 8, ce dispositif auxiliaire 9 est relié à plusieurs autres électrodes auxiliaires 3 dans l'exemple représenté référencées 13, 14 et 15 qui sont collées en des endroits prédéterminés du buste du patient.

Ce dispositif auxiliaire 9 comporte un circuit électronique dont la structure et le fonctionnement seront décrits plus bas.

L'appareil selon l'invention peut être utilisé de plusieurs façons. Selon un premier type de fonctionnement, il est porté en permanence par le patient, l'électrode 2 étant en contact permanent avec la nuque de ce dernier et lors de l'apparition d'un malaise, le patient appuie le boîtier sur sa poitrine ce qui déclenche l'enregistrement d'un électrocardiogramme qui sera ensuite interprété par le médecin spécialiste. Le dispositif électronique de traitement comporte une mémoire qui peut mémoriser plusieurs enregistrements d'une durée déterminée, la durée et le nombre de ces enregistrements pouvant être déterminés par le médecin. Les enregistrements obtenus et mémorisé peuvent être lus directement à partir du boîtier 3 par le médecin lors de la consultation du patient; on peut également prévoir des moyens de transmission, par exemple au moyen du réseau téléphonique, qui permettent d'envoyer les enregistrements qui ont été mémorisés au médecin sans attendre la consultation. Le déclenchement de l'enregistrement d'un électrocardiogramme est obtenu au moyen d'un organe de commande tel qu'un bouton poussoir disposé sur la face extérieure du boîtier 3. Ce bouton poussoir 16 est disposé de préférence sans faire de saillie sur la face extérieure du boîtier 3, de manière à éviter tout déclenchement intempestif.

Selon un autre mode d'utilisation de l'appareil selon l'invention, le boîtier 3 est relié à une ou plusieurs électrodes auxiliaires telles que les électrodes 7, 13, 14 et 15, avec éventuellement l'interposition du dispositif auxiliaire 9 et l'appareil est en état de veille ou de "sentinelle", c'est-à-dire qu'il enregistre en permanence les électrocardiogrammes dans une mémoire tournante du dispositif électronique de traitement et l'actionnement du bouton 16 entraîne l'enregistrement en mémoire fixe d'un électrocardiogramme correspondant à une période s'étendant avant et après le déclenchement; cela permet d'obtenir un électrocardiogramme qui correspond à l'instant d'apparition du malaise et même quelques instants auparavant, ce qui permet d'établir un diagnostic plus précis pour le médecin. Ce mode de fonctionnement qui correspond à la méthode de HOLTER ne présente pas du tout les inconvénients de cette méthode puisque l'appareil est très léger et se supporte très facilement. Il peut donc être porté pendant plusieurs jours sans problème.

Selon une troisième possibilité d'utilisation de l'appareil selon l'invention, les signaux enregistrés sont traités pour obtenir le rythme cardiaque de l'utilisateur et l'on peut alors avantageusement, prévoir des seuils hauts et bas dont le dépassement déclenche une alarme sonore et/ou visuelle. Cette utilisation est particulièrement intéressante pour les malades en rééducation qui doivent veiller à ne pas dépasser un certain rythme cardiaque. Ce mode d'utilisation est également intéressant pour d'autres utilisateurs tels que des sportifs qui peuvent contrôler leur rythme cardiaque au cours d'un entraînement sans avoir à s'arrêter pour prendre leur pulsation cardaque. Cette possibilité est également intéressante pour les plongeurs sous-marins qui doivent également surveiller leur rythme cardiaque et dans ce cas, l'alarme est de préférence lumineuse.

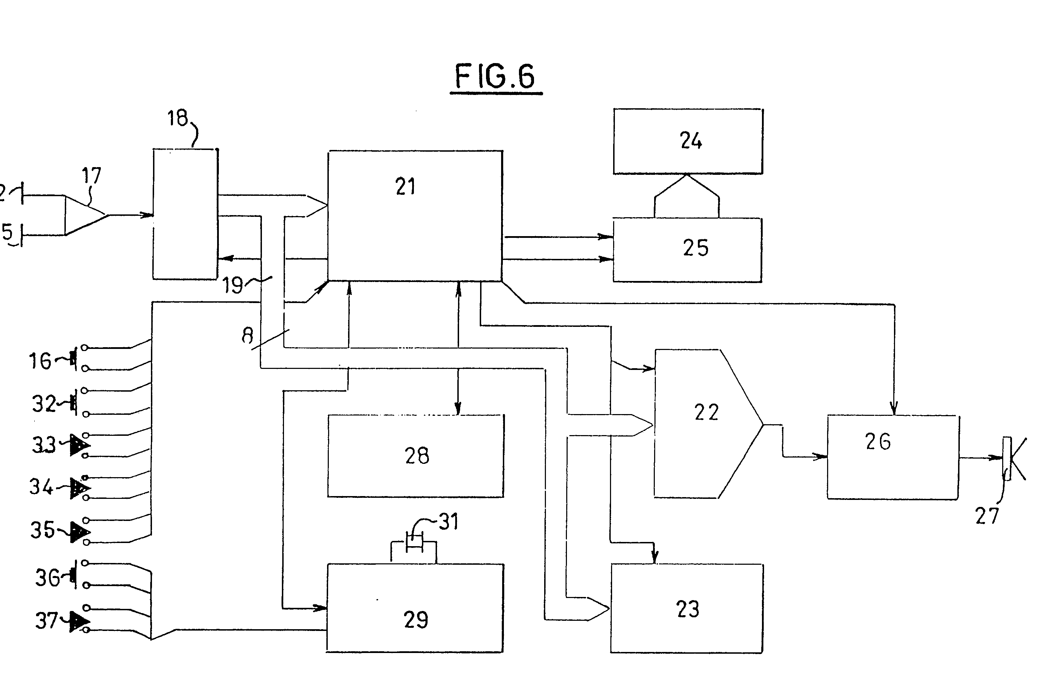

La fig. 6 représente le schéma-bloc d'un mode de réalisation du dispositif électronique de traitement qui est enfermé dans le boîtier 3. Le signal fourni par les électrodes 2 et 5 est d'abord envoyé sur un amplificateur différentiel 17 à haute impédance d'entrée dont les signaux de sortie sont convertis sous forme numérique par un convertisseur analogique-numérique 18. Ce convertisseur 18 est par exemple un convertisseur à huit bits et travaille à un rythme de 100 échantillons par seconde.

Les signaux à huit bits obtenus sont envoyés, par l'intermédiaire d'un bus 19, à une unité logique de commande et de traitement 21, à un convertisseur numérique-analogique 22 et à une mémoire 23 qui est avantageusement constituée par la mémoire vive (RAM) de deux kilo-octets. Un dispositif d'affichage 24, qui est par exemple du type à cristaux liquides, est commandé par une unité de commande d'affichage 25.

Le dispositif électronique de traitement comporte un dispositif de transmission des enregistrements effectués; dans l'exemple représenté, ce dispositif de transmission est du type acoustique et il est essentiellement constitué par un oscillateur commandé par la tension (VCO) 26 qui reçoit les signaux analogiques fournis par le convertisseur 25 et dont le signal de sortie assure l'excitation d'un transducteur acoustique 27 constitué par exemple par un piézo-transducteur.

Le dispositif de traitement électronique comprend également un circuit 28 qui sert au calcul du rythme cardiaque pendant l'enregistrement de l'électrocardiogramme, par exemple en effectuant un calcul récurrent du rythme cardiaque par la détection du segment QRS de l'électrocardio-

gramme. L'appareil peut également comporter une horloge 29 en temps réel qui est par exemple pilotée par un quartz 31.

Le dispositif électronique de traitement est commandé par un certain nombre de boutons ou de poussoirs parmi lesquels on retrouve le bouton-poussoir 16 de commande de l'enregistrement d'un électrocardiogramme, un bouton 32 de commande de l'affichage du rythme cardiaque, deux poussoirs 33 et 34 servant au réglage des seuils haut et bas du rythme cardiaque, un poussoir 35 permettant le réglage de la durée d'un enregistrement d'électrocardiogramme, un bouton 36 commandant l'affichage de l'heure et un poussoir 37 permettant la mise à l'heure de l'horloge.

L'ensemble du dispositif électronique de traitement est réalisé sous forme de circuit intégré et occupe un volume très réduit. Ainsi, dans un exemple de réalisation de l'invention, le boîtier 3 a les dimensions suivantes: longueur: 60 mm, largeur: 40 mm; épaisseur: 18 mm et pèse seulement 100 g.

Dans le premier mode de fonctionnement qui a été décrit plus haut, l'action sur le bouton 16 entraîne le fonctionnement de l'appareil et par suite, l'enregistrement à partir de l'instant d'actionnement de ce bouton 16 d'un électrocardiogramme dont la durée peut être réglée par le médecin traitant au moyen du poussoir 35.

Dans le second mode de fonctionnement, l'électrode 2 est remplacée par l'électrode auxiliaire 7 ou par l'ensemble des électrodes auxiliaires 7, 13, 14, 15 et 16, par l'intermédiaire du dispositif auxiliaire 9; le dispositif électronique enregistre en permanence l'électrocardiogramme et lors de l'actionnement du bouton 16, l'unité de commande 21 commande la mémorisation d'un électrocardiogramme correspondant à une période s'étendant avant et après le déclenchement du bouton 16, par exemple 20 secondes avant et 20 secondes après, la durée de cet enregistrement étant toujours réglée au moyen du poussoir 35.

Les enregistrements mémorisés sous forme numérique dans la mémoire 23 peuvent être lus par le médecin en son cabinet ou transmis par voie téléphonique par l'intermédiaire du circuit 26 et du transducteur 27 après mise sous forme analogique par le convertisseur 22.

Dans le troisième mode de fonctionnement, l'appareil est également en position de veille, c'est-à-dire que le boîtier 3 est relié à l'électrode auxiliaire 7 et on effectue un affichage du rythme cardiaque calculé par le circuit 28. Lorsque ce rythme dépasse les valeurs préréglées au moyen des poussoirs 33 et 34, une alarme sonore et/ou visuelle se produit.

Pour ce dernier mode d'utilisation, on peut également concevoir un appareil simplifié qui ne comporte pas de mémoire ni de dispositif de transmission. Un tel appareil est plus particulièrement destiné aux utilisateurs tels que des sportifs.

La fig. 7 représente le schéma synoptique du circuit compris dans le dispositif auxiliaire 9. Ce circuit comporte essentiellement un multiplexeur 41 qui est branché à l'électrode auxiliaire 7 qui est directement fixée sous le dispositif auxiliaire 9, ainsi qu'aux électrodes auxiliaires 13, 14 et 15 qui sont également de préférence des électrodes de type ambulatoire et qui sont disposées par exemple comme indiqué sur la fig. 8, à savoir deux électrodes 13, 14 en haut de la poitrine du patient, et une électrode 15 au voisinage du coeur. La sortie du multiplexeur comprend une première ligne à une seule voie 42 qui est reliée directement à l'élément d'accouplement et de liaison électrique mâle 11 et une deuxième sortie à deux voies 43 qui est envoyée sur un dispositif de traitement de signal 44 dont la sortie est également envoyée sur l'élément de raccordement mâle 11. Le multiplexeur 41 et l'élément de calcul 44 sont commandés par un circuit de commande 45.

En fonction des instructions fournies par l'unité de commande 45, le multiplexeur 41 soit relie une des électrodes auxiliaires 7, 13, 14 ou 15 à une des entrées de l'amplificateur 17 de manière à obtenir un signal combiné entre l'électrode extérieure principale 2 et une des électrodes auxiliaires, soit relie deux des électrodes auxiliaires au circuit de calcul 44 qui élabore un signal combiné qui est lui-même envoyé au dispositif électronique de traitement du boîtier 3. De cette manière on peut réaliser différents courbes d'électrocardiogrammes correspondant à la combinaison de deux des électrodes 2, 7, 13, 14 et 15. On peut ainsi obtenir un électrocardiogramme qui correspond à ceux qui sont réalisés au moyen d'équipements fixes. Ces enregistrements sont obtenus lorsque l'appareil selon l'invention est en état de "sentinelle", le circuit de commande 45 commandant l'envoi en séquence des différentes combinaisons d'électrodes au dispositif électronique de traitement du boîtier 3.

On voit que l'invention permet d'obtenir très facilement un enregistrement d'électrocardiogrammes en toute circonstance, le maniement de l'appareil étant très simple, l'enregistrement obtenu est de bonne qualité et peut même être du niveau des enregistrements obtenus avec des appareils d'enregistrement fixes. L'appareil selon l'invention apporte une gêne très réduite à l'utilisateur et il est toujours disponible quel que soit l'habillement de l'utilisateur.

La description ci-dessus d'exemples de réalisation n'a été fournie qu'à titre illustratif et nullement limitatif et il est évident que l'on peut y apporter des modifications ou variantes sans pour autant sortir du cadre de la présente invention. En particulier, comme cela a été indiqué plus haut, on peut prévoir d'autres dispositifs de maintien ou de suspension des électrodes tels que des harnais ou des bandoulières. Par ailleurs, le lien 1 peut être partiellement spiralé, la partie torsadé en ressort s'étendant approximativement entre la clavicule et quelques centimètres avant le boîtier.

## Revendications

1. Appareil portatif de surveillance de l'activité cardiaque, comportant deux électrodes branchées dans un boîtier (3) renfermant un dispositif électronique de traitement des signaux enregistrés à partir de l'activité diastolique et systolique du coeur, les dites électrodes (2, 5) étant séparées et affectées à deux parties distinctes de la région cardiaque du buste du patient, l'une desdites électrodes (5) étant apparente sur l'une des faces (4) du boîtier (3) pour entrer en contact avec une première zone du buste du patient tandis que l'autre électrode (2) est indépendante de ce même boîtier pour entrer en contact avec une seconde zone dudit buste, ladite autre électrode (2) étant branchée électriquement dans ledit boîtier (3) par un conducteur de liaison (1), caractérisé en ce que ledit conducteur de liaison (1) est constitué d'un lien souple en forme de boucle qui sert simultanément pour la suspension de l'appareil sur le corps du patient, ledit boîtier (3) et ladite électrode (2) étant fixés en des points sensiblement opposés de ladite boucle.

2. Appareil selon la revendication 1, caractérisé en ce que le boîtier (3) comprend au moins un organe d'accouplement mécanique et électrique (6) avec une électrode auxiliaire (7) fixée sur la peau du patient.

3. Appareil selon l'une des revendications 1 ou 2, caractérisé en ce que l'électrode (2) indépendante du boîtier (3) comporte un organe d'accouplement mécanique et électrique (6) avec une électrode auxiliaire fixée sur la peau du patient.

4. Appareil selon l'une quelconque des revendications 1 à 3, caractérisé en ce que les deux électrodes (2, 5) sont souples et déformables et sont réalisées en une matière conductrice du type à mailles ou à tresses.

5. Appareil selon la revendication 2, caractérisé en ce qu'il comporte un dispositif auxiliaire (9) en forme de boîtier plat comportant sur une de ses faces un dispositif d'accouplement mâle (11) du type précité et sur la face opposée un dispositif d'accouplement femelle (12), ledit dispositif auxiliaire (9) étant par ailleurs relié à des électrodes (13, 14, 15) collées en permanence sur différents endroits du buste du patient.

6. Appareil selon l'une quelconque des revendications 1 à 5, caractérisé en ce qu'il comporte un convertisseur analogique-numérique et une mémoire, et en ce que le boîtier (3) comporte un bouton de commande (16) dont l'actionnement commande l'enregistrement d'un électrocardiogramme dans ladite mémoire.

7. Appareil selon la revendication 6, caractérisé en ce que le dispositif électronique de traitement des signaux comporte une mémoire tournante et en ce que l'actionnement du bouton de commande (16) commande l'enregistrement d'un électrocardiogramme correspondant à une période s'étendant avant et après dudit bouton (16).

8. Appareil selon l'une quelconque des revendications 1 à 7, caractérisé en ce que le dispositif électronique de traitement des signaux comporte un circuit de calcul du rythme cardiaque et en ce qu'il comporte des éléments de réglage de seuils du rythme cardiaque dont le franchissement déclenche une alarme.

9. Appareil selon l'une quelconque des revendications 1 à 8, caractérisé en ce que les électrodes auxiliaires sont encollées sur leurs deux faces.

10. Appareil selon l'une quelconque des revendications 1 à 9, caractérisé en ce qu'il comporte un dispositif de transmission acoustique des électrocardiogrammes enregistrés.

11. Appareil selon la revendication 5, caractérisé en ce que le dispositif auxiliaire (9) comprend un multiplexeur et un circuit de traitement de signaux.

## Patentansprüche

1. Tragbares Gerät zur Überwachung der Herztätigkeit des Typs, der zwei an einem Gehäuse (3) angeschlossene Elektroden aufweist, welches eine elektronische Einrichtung zur Verarbeitung von Signalen einschließt, die ausgehend von der diastolischen und der systolischen Herzaktivität aufgenommen wurden, wobei die beiden Elektroden (2, 5) voneinander getrennt und an zwei bestimmten Bereichen des Herzbereichs des Patientenoberkörpers angeordnet sind, wobei die eine der Elektroden auf einer der Oberflächen (4) des Gehäuses (3) liegt, um in Berührung mit einer ersten Zone des Patientenoberkörpers zu treten, während die andere Elektrode (2) unabhängig von diesem Gehäuse ist, um in Berührung mit einer zweiten Zone des Oberkörpers zu treten und wobei die genannte andere Elektrode (2) elektrisch mittels eines Verbindungsleiters (1) mit dem Gehäuse (3) verbunden ist, dadurch gekennzeichnet, daß der Verbindungsleiter (1) aus einer nachgiebigen Verbindung in Schlingenform besteht, die gleichzeitig zum Tragen des Gerätes am Patientenkörper dient, wobei das Gehäuse (3) und die Elektrode (2) an Stellen der Schleife befestigt sind, die einander im wesentlichen gegenüberliegen.

2. Gerät nach Anspruch 1, dadurch gekennzeichnet, daß das Gehäuse (3) wenigstens ein mechanisches und elektrisches Ankupplungselement (6) mit einer auf der Haut des Patienten befestigten Hilfselektrode (7) umfaßt.

3. Gerät nach Anspruch 1 oder 2, dadurch ge-

EP 0 147 253 B1

kennzeichnet, daß die vom Gehäuse (3) unabhängige Elektrode (2) ein mechanisches und elektrisches Ankupplungselement (6) mit einer auf der Haut des Patienten befestigten Hilfselektrode umfaßt.

4. Gerät nach einem der Ansprüche 1 bis 3, dadurch gekennzeichnet, daß die beiden Elektroden (2, 5) nachgiebig und verformbar sind und aus einem leitfähigen Maschen- oder Geflechtmaterial ausgebildet sind.

5. Gerät nach Anspruch 2, dadurch gekennzeichnet, daß das Gerät eine Zusatzeinrichtung (9) in Form eines flachen Gehäuses aufweist, das auf einer seiner Oberflächen ein männliches Ankupplungselement (11) der vorgenannten Art und auf seiner gegenüberliegenden Außenseite ein weibliches Ankupplungselement (12) aufweist, wobei die Zusatzeinrichtung (9) außerdem mit Elektroden (13, 14, 15) verbunden ist, die bleibend an verschieden Orten des Patientenoberkörpers aufgeklebt sind.

6. Gerät nach einem der Ansprüche 1 bis 5, dadurch gekennzeichnet, daß das Gerät einen numerischen Analogwandler und einen Speicher umfaßt und daß das Gehäuse (3) einen Betätigungsknopf (16) trägt, bei dessen Betätigung die Aufzeichnung eines Elektrokardiogramms in dem genannten Speicher ausgelöst wird.

7. Gerät nach Anspruch 6, dadurch gekennzeichnet, daß die elektronische Einrichtung zur Signalverarbeitung einen Umlaufspeicher enthält und daß die Betätigung des Betätigungsknopfes (16) die Aufzeichnung eines Elektrokardiogramms auslöst, das einem Zeitraum entspricht, dar sich vor und nach der Betätigung des Knopfes (16) erstreckt.

8. Gerät nach einem der Ansprüche 1 bis 7, dadurch gekennzeichnet, daß die elektronische Einrichtung zur Signalverarbeitung eine Schaltung zur Berechnung des Herzrhythmus enthält und daß sie Schwellwert-Steuereinrichtungen für den Herzrhythmus enthält, deren Überschreitung einen Alarm auslöst.

9. Gerät nach einem dar Ansprüche 1 bis 8, dadurch gekennzeichnet, daß die Hilfselektroden auf ihren beiden Oberflächen mit Klebstoff versehen sind.

10. Gerät nach einem der Ansprüche 1 bis 9, dadurch gekennzeichnet, daß das Gerät eine Einrichtung zur akustischen Übertragung der aufgezeichneten Elektrokardiogramme enthält.

11. Gerät nach Anspruch 5, dadurch gekennzeichnet, daß die Zusatzeinrichtung (9) einen Multiplexer und Signalverarbeitungsschaltung enthält.

**Claims**

1. Portable apparatus for monitoring cardiac activity, of the type comprising two electrodes connected into a housing (3) which contains an electronic device for processing signals recorded from the diastolic and systolic activity of the heart, the said electrodes (2, 5) being separated and being allocated to two different parts of the cardiac region of the patient's chest, one of the said electrodes (5) being situated on one of the faces (4) of the housing (3) in such a manner as to come in contact with a first zone of the patient's chest, whereas the other electrode (2) is independent of this same housing, in such a manner as to come in contact with a second zone of the said chest, the said other electrode (2) being electrically connected-into the said housing (3) by means of a conducting connector (1), characterised in that the said conducting connector (1) is constituted by a flexible lead which is in the form of a loop which serves simultaneously for the suspension of the apparatus on the patient's body, the said housing (3) and the said electrode (2) being fixed at substantially opposite points of the said loop.

2. Apparatus according to claim 1, characterised in that the housing (3) comprises at least one mechanical and electrical member (6) for coupling with an auxiliary electrode (7) fixed on the patient's skin.

3. Apparatus according to any one of the claims 1 or 2, characterised in that the electrode (2) which is independent of the housing (3) comprises a mechanical and electrical member (6) for coupling with an auxiliary electrode fixed on the patient's skin.

4. Apparatus according to any one of the claims 1 to 3, characterized in that the two electrodes (2, 5) are flexible and deformable and are made from a conductive material of the meshed or braided type.

5. Apparatus according to claim 2, characterised in that it comprises an auxiliary device (9) in the form of a flat housing comprising on one of its faces a male coupling device (11) of the aforementioned type, and on the opposite face, a female coupling device (12), the said auxiliary device (9) furthermore being connected to electrodes (13, 14, 15) which are permanently glued onto different places on the patient's chest.

6. Apparatus according to any one of the claims 1 to 5, characterised in that it comprises an analogue-digital converter and a memory, and in that the housing (3) comprises a control button (16) whose actuation controls the recording of an electro-cardiogram in the said memory.

7. Apparatus according to claim 6, characterised in chat the electronic device for processing signals comprises a rotating memory, and in that the actuation of the control button (16) controls the recording of an electro-cardiogram corresponding to a period extending before and after the said button (16) [sic].

8. Apparatus according to any one of the claims 1 to 7, characterised in that the electronic device for processing signals comprises a circuit for calculating the cardiac rhythm, and in that it comprises elements for regulating the thresholds of the cardiac rhythm, and sets off an alarm when these thresholds arc exceeded.

9. Apparatus according to any one of the claims 1 to 8, characterised in that the auxiliary electrodes are coated with glue on their two faces.

10. Apparatus according to any one of the claims 1 to 9, characterised in that it comprises a device for acoustic transmission of the electro-cardiograms recorded.

11. Apparatus according to claim 5, characterised in that the auxiliary device (9) comprises a multiplexer and a circuit for processing signals.

FIG.1

FIG.2

FIG.3

FIG.4

1

EP 0 147 253 B1

## FIG.5

## FIG.7

## FIG.8

3

FIG.6